# EUROPEAN PATENT SPECIFICATION

(11) **EP 1 838 659 B1**
(45) Date of publication and mention of the grant of the patent: **15.07.2015**
(21) Application number: 05824316.3
(22) Date of filing: 22.12.2005
(51) Int. Cl.: C07C 213/02, C07C 309/65, C07C 309/73

(54) **PROCESS FOR PREPARATION OF N,N-DIISOPROPYL-3-(2-HYDROXY-5-METHYLPHENYL)-3-PHENYLPROPYLAMINE**
VERFAHREN ZUR HERSTELLUNG VON N,N-DIISOPROPYL-3-(2-HYDROXY-5-METHYLPHENYL)-3-PHENYLPROPYLAMIN
PROCEDE DE PREPARATION DE N,N-DIISOPROPYL-3-(2-HYDROXY-5-METHYLPHENYL)-3-PHENYLPROPYLAMINE

(30) Priority: 24.12.2004 SI 200400349
(43) Date of publication of application: 03.10.2007
(73) Proprietor: LEK Pharmaceuticals d.d., 1526 Ljubljana (SI)
(72) Inventor: TOPLAK, Renata, 2000 Maribor (SI)
(74) Representative: Oser, Andreas
(86) International application number: PCT/EP2005/013899
(87) International publication number: WO 2006/066931

(56) References cited:
- WO-A-03/035599
- WO-A-2005/061432
- US-A1- 2003 027 856
- DATABASE CAPLUS [Online] CHEMICAL ABSTRACTS SERVICE, COLUMBUS, OHIO, US; LUSTIG, PETR ET AL: "Process for preparing N,N-diisopropyl-3-(2-hydroxy-5-methylpheny l)-3- phenylpropylamine in racemic or optically active form thereof" XP002376934 retrieved from STN Database accession no. 2005:41309 -& CZ 293 791 B6 (ZENTIVA, A.S., CZECH REP.) 14 July 2004 (2004-07-14)
- DATABASE CAPLUS [Online] CHEMICAL ABSTRACTS SERVICE, COLUMBUS, OHIO, US; ZHAO, ZHIQUAN: "Method for preparation of tolterodine tartrate" XP002376935 retrieved from STN Database accession no. 2006:80185 & CN 1 626 504 CN (LUNAN PHARMACEUTICAL CO., LTD., PEOP. REP. CHINA) January 2004 (2004-01)

## Description

### Technical Field

The invention belongs to the field of organic chemistry and relates to a novel efficient synthetic process for the preparation of N,N-diisopropyl-3-(2-hydroxy-5-methylphenyl)-3-phenylpropylamine characterized by an easily obtainable 3-(2-hydroxy-5-methylphenyl)-3-phenylpropanol as a key intermediate.

### Background Art

Several synthetic approaches for preparation of N,N-diisopropyl-3-(2-hydroxy-5-methylphenyl)-3-phenylpropylamine have been described. Prior art literature describes preparation of racemic and enantiomerically pure product, which is obtained either with the resolution of the enantiomers in the last step or the chiral synthesis.

EP 325571 reveals a multiple step process for the preparation of N,N-diisopropyl-3-(2-hydroxy-5-methylphenyl)-3-phenylpropylamine starting from 4-(-methoxy-5-methylphenyl)-6-methyl-3,4-dihydrocoumarin as the first intermediate. Lactone ring opening in the basic medium with simultaneous esterification of carboxylic group and etherification of phenol hydroxyl leads to 3,3-diphenylpropionic acid ester. Subsequent reduction of the propionate intermediate, tosylation of alcohol moiety, substitution with diisopropylamine and deprotection of the phenol group yields N,N-diisopropyl-3-(2-hydroxy-5-methylphenyl)-3-phenylpropylamine Resolution of the enantiomers is achieved by fractional crystallisation using (+)-L-tartaric acid resulting in pure chiral crystalline tartarate. Also WO 03/01460 addresses the above process.

According to WO 04/078700 the cleavage of the methyl ether protecting group from the phenol moiety by heating in a mixture of aqueous HBr and acetic acid facilitates the formation of the N,N-diisopropyl-3-(2-hydroxy-5-methylphenyl)-3-phenylpropylamine dimer.

US 5922914 teaches a four reaction steps process using 3,4-dihydro-6-methyl-4-phenyl-2-benzopyran-2-ol as a key intermediate. Amination with diisopropylamine proceeds in the presence of hydrogen at elevated pressure.

CZ 293791 discloses a process for preparing N,N-diisopropyl-3-(2-hydroxy-5-methylphenyl)-3-phenylpropylamine comprising the following steps: a) reductive lactone ring opening of 3,4-dihydro-6-methyl-4-phenyl-2-benzopyran-2-one to yield 3-(2-hydroxy-5-methylphenyl)-3-phenylpropanol; b) transforming both hydroxy groups of the compound obtained in previous step with the same reagent to form the ditosyl, dimesyl or dicamphor-10-sulphonyl derivative; c) substituting the sulphonyl substitutent on the propyl chain of the compound obtained in previous step with diisopropylamine; and d) hydrolysing the compound obtained in previous step to transform the remaining sulphonyl substitutent into an hydroxy substituent.

J There are known several approaches of enantioselective synthesis of enantiomers of 3-(2-hydroxy-5-methylphenyl)-N,N-diisopropyl-3-phenylpropylamine.

Multiple step process for the preparation of enantiomerically enriched (+)-R-3-(2-hydroxy-5-methylphenyl)-N,N-diisopropyl-3-phenylpropyfamine relies on the synthesis of the enantiomerically enriched 3,4-dihydro-6-methyl-4-phenyl-2-benzopyran-2-one as disclosed in US 6310248. Enantiomerically enriched benzopyran-2-one intermediate is prepared by the 3-step process starting with enantioselective reduction of carbonyl group of 3-methyl-3-phenyl-1-inden-1-on followed by the sigmatropic rearrangement and Bayer-Villiger oxidation.

US 6410746 reveals application of the bis-transitional metal (rhodium) catalyst for the insertion reaction in the preparation of enantiomerically enriched gemdiarylalkyl derivatives.

Asymmetric synthesis of enantiomers of 3-(2-hydroxy-5-methylphenyl)-N,N-diisopropyl-3-phenylpropylamine was published in J. Organic Chem., 1998, 63, 8067 and comprises copper-assisted asymmetric conjugate addition of aryl Grignard reagent to phenylpropenoyl derivative of oxazolidinone used as a chiral auxiliary. Another approach was published in the Organic Process Research and Development (2002, 6, 379)

3,4-dihydro-6-methyl-4-phenyl-2H-benzopyran-2-one is a known from WO 01/49649 and its use in a synthesis of a title compound is known from CZ 293791.

Despite several described approaches towards N,N-diisopropyl-3-(2-hydroxy-5-methylphenyl)-3-phenylpropylamine a need for shorter, less expensive and more industrially applicable processes performed under milder conditions still remains. Namely, N,N-diisopropyl-3-(2-hydroxy-5-methylphenyl)-3-phenylpropylamine prepared from 3,4-dihydro-6-methyl-4-phenyl-2H-benzopyranon is a key starting compound for the preparation of tolterodine, (+)-(R)-3-(2-hydroxy-5-methylphenyl)-N,N-diisopropyl-3-phenylpropylamine, currently marketed as (+)-L-tartarate salt - an important urological drug, which acts as a nonsubtype selective muscarinic receptor antagonist. It is used for manufacturing of the medicament for treating the patients with overactive bladder showing symptoms of urinary frequency, urgency, or urge incontinence and can be used for treating asthma, CODP and allergic rhinitis. Also its metabolite a 5-hydroxymethyl exhibits antimuscarinic activity.

### Disclosure of the Invention

The present invention provides process for preparation of N,N-diisopropyl-3-(2-hydroxy-5-methylphenyl)-3-phenylpropylamine or a salt thereof, as defined in anyone of claims 1 to 3 and 9. The present invention also provides a compound according to claim 15. Preferred embodiments are set forth in the subclaims.

In an aspect the invention is a new process for preparation of N,N-diisopropyl-3-(2-hydroxy-5-methylphenyl)-3-phenylpropylamine or a salt thereof from 3,4-dihydro-6-methyl-4-phenyl-2-benzopyran-2-one which proceeds via novel intermediates, the first of them being 3-(2-hydroxy-5-methylphenyl)-3-phenylpropanol. Those intermediates which are used in the process are compounds of formula: 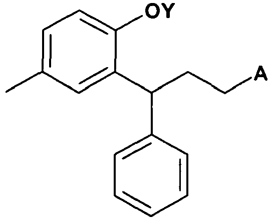 where Y can be: H, or Y can be: COR where R is selected from C₁-C₃ alkyl NMe₂; or Y can be: Ar-SO₂ where Ar is R'-C₆H₄ where R' is selected from H, halogen, NO₂; or Y can be: R"- SO₂- where R" is selected from C₂-C₄ alkyl, fluorinated C₁-C₄ alkyl, halogen, NR"'₂(CH₂), where each R'" is independently selected from C₁-C₃ alkyl, and NMe₃(CH₂)⁺; and A, which is the substituent on the propyl chain, can be OY; or A can be I or Br;
or A can be NR₁R₂ where R₁ and R₂ can be same or different selected from H or C₁-C₃ alkyl, with proviso that if Y is H, than R₁ and R₂ must contain together less than three carbon atoms

Thus in an aspect the invention is a process characterized in that the 3-(2-hydroxy-5-methylphenyl)-3-phenylpropanol is transformed into compound of formula: 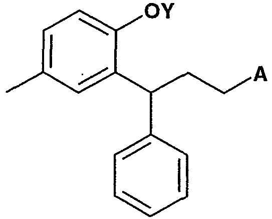 where Y and A are as described above, preferably A is OY, further characterized in that substituent A is subsequently transformed into I or Br; and into N(i-Pr)₂. Specifically in an aspect 3-(2-hydroxy-5-methylphenyl)-3-phenylpropanol is prepared from 3,4-dihydro-6-methyl-4-phenyl-2-benzopyran-2-one, and specifically in one aspect in one of the steps the mixture of enantiomers, preferably in last step the mixture of enantiomers of N,N-diisopropyl-3-(2-hydroxy-5-methylphenyl)-3-phenylpropylamine is resolved.

The process embodied in our invention consisting of the following steps:
a) reductive lactone ring opening of 3,4-dihydro-6-methyl-4-phenyl-2-benzopyran-2-one to yield 3-(2-hydroxy-5-methylphenyl)-3-phenylpropanol;
b) transforming both hydroxy groups of the compound obtained in previous step with the same reagent to form di-O-substituted derivative, that is characterized in that the O-substituent on the propyl chain reacts more easily with diisopropylamine than O-substituent on the aromatic ring;
c) substituting the O-substitutent on the propyl chain of the compound obtained in previous step with a halogen;
d) substituting the halogen on the propyl chain of the compound obtained in previous step with an amine;
e) hydrolysing the compound obtained in previous step to transform the remaining O-substitutent into hydroxy substituent; and
f) optionally optically resolving the mixture of enantiomers obtained in any of the previous steps.

In specific embodiment, the process comprises the following sequence of steps:
a) reductive lactone ring opening of 3,4-dihydro-6-methyl-4-phenyl-2-benzopyran-2-one to yield 3-(2-hydroxy-5-methylphenyl)-3-phenylpropanol;
b) esterifying both hydroxy groups with the same acid or acid derivative, so that each O-substituent is an ester group, characterized in that the formed ester group on the propyl chain reacts more easily with diisopropylamine than the ester group on the aromatic ring;
c) substituting the ester group on the propyl chain with iodine;
d) substituting the halogen which is iodine on the propyl chain with an amine which is diisopropylamine;
e) hydrolysing the remaining ester group, and
f) optionally optically resolving the mixture of enantiomers obtained in any of the steps, wherein either steps c) and d) are performed as a combined step or steps b) to d) are performed in a single pot.

In another specific aspect of the process the hydroxy groups of 3-(2-hydroxy-5-methylphenyl)-3-phenylpropanol are esterified with an acid, or a halide or anhydride of an acid selected from group consisting of: benzensulfonic acid, 4-bromobenzenesulfonic acid, 4-nitrobenzenesulfonic acid, ethanesulfonic acid, propanesulfonic acid, butansulfonic acid, trifluoromethanesulfonic acid, 2,2,2-trifluoroethanesulfonic acid, nonafluorobutanesulfonic acid, and fluorosulfonic acid, preferably benzensulfonic acid or ethanesulfonic acid in the presence of an organic base which is preferably pyridine, substituted pyridine or tertiary amine.

In another specific aspect of the process the O-substitutent on the propyl chain of the compound obtained is substituted with an amine in presence of a halide, preferably sodium iodide.

Specific embodiment of the invention is a compound (+)-N,N-diisopropyl-3-(2-hydroxy-5-methylphenyl)-3-phenylpropylamine hydrogen tartrate prepared from N,N-diisopropyl-3-(2-hydroxy-5-methylphenyl)-3-phenylpropylamine obtained according to the any of the described processes and a pharmaceutical composition comprising said compound.
New compounds of formula: 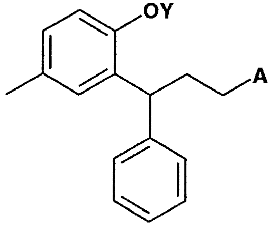 where Y can be: H, or Y can be: COR where R is selected from C₁-C₃ alkyl or Y can be: P(OR)₂ where R is selected from C₁-C₃ alkyl; or Y can be: PX(Z)₂ where X is selected from O, N-SO₂-C₆H₄-Me, NPh; and Z is selected from OPh, NMe₂; or Y can be: Ar-SO₂ where Ar is R-C₆H₄ where R' is selected from H, halogen, NO₂; or Y can be: R"- SO₂- where R" is selected from C₂-C₄ alkyl, fluorinated C₁-C₄ alkyl, halogen, NMe₃(CH₂)⁺ ;
and A is OY or A is I or Br; or A is NR₁R₂ where R₁ and R₂ can be same or different selected from H or C₁-C₃ alkyl, with proviso that if Y is H, than R₁ and R₂ must contain together less than three carbon atoms, presents specific embodiments of the invention.

Preferred specific embodiments of the invention are (2-(benzenesulphonyloxy)-5-methylphenyl)-3-phenylpropyl-p-benzenesulphonate; 3-(2-ethanesulphonyloxy-5-methylphenyl)-3-phenylpropyl-ethanesulphonate; N,N-diisopropyl-3-(2-( benzenesulphonyloxy)-5-methylphenyl)-3-phenylpropyl amine, N,N-diisopropyl-3-(2-ethanesulphonyloxy-5-methylphenyl)-3-phenylpropyl amine, 3-(2-( benzenesulphonyloxy)-5-methylphenyl)-3-phenylpropyl iodide, and 3-(2-ethanesulphonyloxy-5-methylphenyl)-3-phenylpropyl iodide as well as 3-(2-( benzenesulphonyloxy)-5-methylphenyl)-3-phenylpropyl iodide and bromide, and 3-(2-ethanesulphonyloxy-5-methylphenyl)-3-phenylpropyl iodide and bromide.

Those compounds may be used in the process of preparing a medicament for treating overactive bladder.

The preferred specific aspect of the invention is the use of sodium iodide in the process of manufacturing N,N-diisopropyl-3-(2-hydroxy-5-methylphenyl)-3-phenylpropyl amine, preferably its use in manufacturing of an intermediate compound of formula 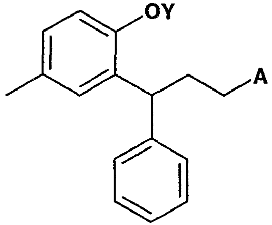 where Y can be: COR where R is selected from C₁-C₃ alkyl
or Y can be: P(OR)₂ where R is selected from C₁-C₃ alkyl; or Y can be: PX(Z)₂ where X is selected from O, N-SO₂-C₆H₄-Me, NPh; and Z is selected from OPh, NMe₂; or Y can be: Ar-SO₂ where Ar is R'-C₆H₄ where R' is selected from H, halogen, NO₂; or Y can be: R"- SO₂- where R" is selected from C₂-C₄ alkyl, fluorinated C₁-C₄ alkyl, halogen, NMe₃(CH₂)⁺ ; and A is NR₁R₂ where R₁ and R₂ can be same or different selected from H or C₁-C₃ alkyl.

### Detailed Description of the Invention

The process of our invention is depicted on Scheme and comprises following steps:
a) reductive lactone ring opening of the lactone ring of optionally substituted compound of Formula I giving 2-hydroxypheylpropanol derivative, a compound represented with Formula **II**, which can be optionally substituted, preferably reductive lactone ring opening of 3,4-dihydro-6-methyl-4-phenyl-2-benzopyran-2-one to yield 3-(2-hydroxy-5-methylphenyl)-3-phenylpropanol;
b) transforming both hydroxy groups of the compound of Formula **II** with the same reagent to form di-O-substituted derivative, which is performed by treatment with a reagent that simultaneously activates the alcohol moiety and protects the phenol moiety. The transformation is characterized in that the O-substituent on the propyl chain reacts more easily with diisopropylamine than O-substituent on the aromatic ring yielding optionally substituted compound of Formula **III**;
c) substituting the O-substituent on the propyl chain with a halogen, preferably iodine, yielding an intermediate compound of Formula **IV** having O-substituent on the aromatic ring and bearing iodine substituent on alkyl chain; and
d) substituting the halogen on the propane chain with an amine, preferably diisopropylamine yielding optionally substituted compound of formula **V** or its analog,
e) hydrolysing the remaining O-substituent giving optionally substituted compound of formula **VI** or its analog; and
f) optionally optically resolving the mixture of enantiomers obtained in any, preferably in previous step. In case the aminating agent used in step d) is different from diisopropylamine the formed analog of optionally substituted compound of formula **V** can be in an intermediate or final step optionally converted to another amine, preferably diisopropylamine.

In the preferred embodiment the process comprises following steps: a) reductive lactone ring opening of 3,4-dihydro-6-methyl-4-phenyl-2-benzopyran-2-one to yield 3-(2-hydroxy-5-methylphenyl)-3-phenylpropanol; b) esterification of both hydroxy groups with an acid derivative which forms with the said hydroxy group an easily substitutable group such as alkylcarboxy, arylcarboxy, sulphonyloxy, phosphoryloxy group attached to propane chain; c) substituting the group on the propyl chain with diisopropylamine in the presence of Nal; d) hydrolyzing the group, which is remained bound to give 3-(2-hydroxy-5-methylphenyl)-N,N-diisopropyl-3-phenylpropylamine; and e) optionally optically resolving the mixture of obtained enantiomers.

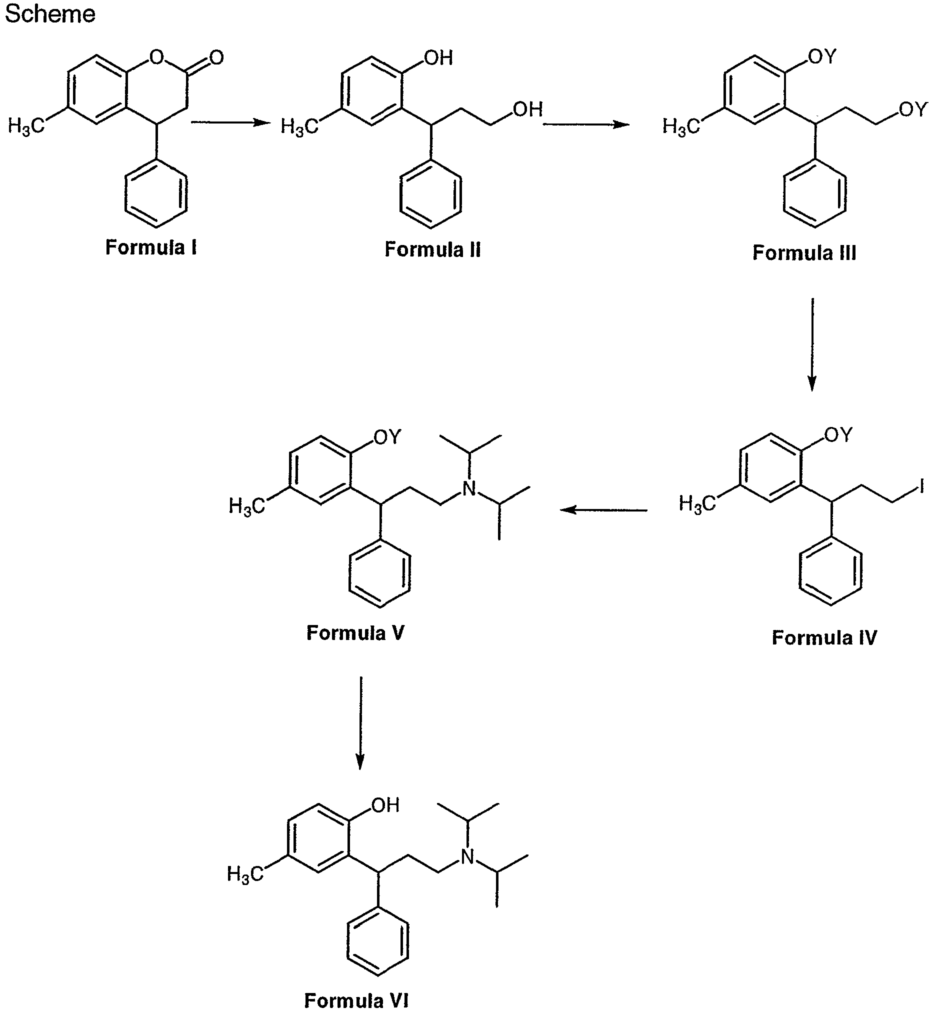

The intermediates of Formula **III** are generally those where Y can be: COR where R is selected from C₁-C₄ alkyl, completely or partially fluorinated C₁-C₃ alkyl or Y can be P(OR)₂ where R is selected from C₁-C₃ alkyl; or Y can be: PX(Z)₂ where X is selected from O, NTs, NPh; and Z is selected from OPh, NMe₂;or Y can be: Ar-SO₂ where Ar is R'-C₆H₄ where R' is selected from H, halogen, NO₂; or Y can be: R"- SO₂- where R" is selected from C₂-C₄ alkyl, preferably C₄H₉; completely or partially fluorinated C₁-C₄ alkyl, preferably CF₃ or C₄F₉ or CF₃-CH₂; halogen, preferably F; NMe₃(CH₂); preferably selected from: 3-(2-(benzenesulphonyloxy)-5-methylphenyl)-3-phenylpropyl-benzenesulphonate, 3-(2-(p-nitrobenzenesulphonyloxy)-5-methylphenyl)-3-phenylpropyl-p-nitrobenzenesulphonate; 3-(2-(p-bromobenzenesulphonyloxy)-5-methylphenyl)-3-phenylpropyl-p-bromobenzenesulphonate;3-(2-triflouromethanesulphonyloxy-5-methylphenyl)-3-phenylpropyl-trifluoromethanesulphonate, 3-(2-(2,2,2-trifluoroethanesulphonyloxy-5-methylphenyl)-3-phenylpropyl-2,2,2-trifluoroethanesulphonate, 3-(2-nonafluorobutanesulphonyloxy-5-methylphenyl)-3-phenylpropyl-nonafluorobutanesulphonate, 3-(2-triflourosulphonyloxy-5-methylphenyl)-3-phenylpropyl-trifluorosulphonate, 3-(10-camphorsulfonyloxy -5-methylphenyl)-3-phenylpropyl-10-camphorsulfonate, 3-(2-acetyloxy-5-methylphenyl)-3-phenylpropyl acetate, 3-(2-trifluoroacetyloxy-5-methylphenyl)-3-phenylpropyl trifluoroacetate, 3-(2-(diphenylphosphoryloxy)-5-methylphenyl)-3-phenylpropyl-diphenylphosphate, 3-(2-(diethylphosphito)-5-methylphenyl)-3-phenylpropyl-diethylphosphite

In the general intermediates of formula **IV** Y is as above, while iodine can generally be replaced by another halogen. Preferably they are selected from: 3-(2-(benzenesulphonyloxy)-5-methylphenyl)-3-phenylpropyl-iodide, 3-(2-(p-nitrobenzenesulphonyloxy)-5-methylphenyl)-3-phenylpropyl- iodide; 3-(2-(p-bromobenzenesulphonyloxy)-5-methylphenyl)-3-phenylpropyl- iodide; 3-(2-triflouromethanesulphonyloxy-5-methylphenyl)-3-phenylpropyl- iodide, 3-(2-(2,2,2-trifluoroethanesulphonyloxy-5-methylphenyl)-3-phenylpropyl- iodide, 3-(2-nonafluorobutanesulphonyloxy-5-methylphenyl)-3-phenylpropyl- iodide, 3-(2-triflourosulphonyloxy-5-methylphenyl)-3-phenylpropyl-trifluorosulphonate, 3-(10-camphorsulfonyloxy -5-methylphenyl)-3-phenylpropyl- iodide, 3-(2-acetyloxy-5-methylphenyl)-3-phenylpropyl iodide, 3-(2-trifluoroacetyloxy-5-methylphenyl)-3-phenylpropyl iodide, 3-(2-(diphenylphosphoryloxy)-5-methylphenyl)-3-phenylpropyl- iodide, 3-(2-(diethylphosphito)-5-methylphenyl)-3-phenylpropyl-iodide or respective bromides.

Intermediates of Formula **V** can generally also have following structure:

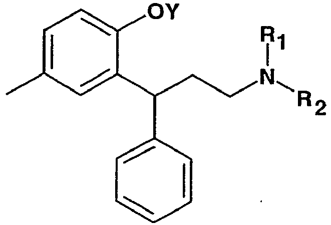

Where Y is as defined above and R₁ and R₂ can be same or different selected from H or optionally substituted C₁-C₄ alkyl, preferably R₁ and R₂ are the same and are i-Pr or when Y is H, R₁ can be H or C₁-C₂ alkyl and R₂ can be H or C₁-C₄ alkyl, and are preferably selected from: N,N-diisopropyl-3-(2-(benzenesulphonyloxy)-5-methylphenyl)-3-phenylpropyl amine, N,N-diisopropyl-3-(2-(p-nitrobenzenesulphonyloxy)-5-methylphenyl)-3-phenylpropyl amine, N,N-diisopropyl-3-(2-(p-bromobenzenesulphonyloxy)-5-methylphenyl)-3-phenylpropyl amine, N,N-diisopropyl-3-(2-trifloromethanesufphonyloxy-5-methylphenyl)-3-phenylpropyl amine, N,N-diisopropyl-3-(2,2,2-trifluoroethanesulphonyloxy-5-methylphenyl)-3-phenylpropyl amine, N,N-diisopropyl-3-(2-nonafluorobutanesulphonyloxy-5-methylphenyl)-3-phenylpropyl amine, N,N-diisopropyl-3-(2-(10-camphorsulfonyloxy)-5-methylphenyl)-3-phenylpropyl amine; N,N-diisopropyl-3-(2-acetyloxy-5-methylphenyl)-3-phenylpropyl amine, N,N-diisopropyl-3-(2-trifluoroacetyloxy-5-methylphenyl)-3-phenylpropyl amine, N,N-diisopropyl-3-(2-diphenylphosphoryloxy -5-methylphenyl)-3-phenylpropyl amine, N,N-diisopropyl-3-(2-diethylphosphito-5-methylphenyl)-3-phenylpropyl amine.

Most preferred intermediates are: 3-(2-(benzenesulphonyloxy)-5-methylphenyl)-3-phenylpropyl benzenesulphonate; 3-(2-ethanesulphonyloxy-5-methylphenyl)-3-phenylpropyl ethanesulphonate; 3-(2-(benzenesulphonyloxy)-5-methylphenyl)-1-iodopropane; 3-(2-methanesulphonyloxy-5-methylphenyl)-3-phenyl-1-iodopropane; 3-(2-ethanesulphonyloxy-5-methylphenyl)-3-phenyl-1- iodopropane; N,N-diisopropyl-3-(2-(benzenesulphonyloxy)-5-methylphenyl)-3-phenylpropyl amine and N,N-diisopropyl-3-(2-ethanesulphonyloxy-5-methylphenyl)-3-phenylpropyl amine.

3,4-Dihydro-6-methyl-4-phenyl-2-benzopyran-2-one (compound of formula **I**) is readily accessible starting material. Surprisingly, we found that it can be transformed to N,N-diisopropyl-3-(2-hydroxy-5-methylphenyl)-3-phenylpropylamine (compound of formula **VI**) by faster and more convenient synthetic approach using less expensive and hazardous chemicals than previously known. The present invention reveals a short process performed under mild reaction conditions and therefore suitable for large scale production.

The reductive opening of the lactone ring of the compound of formula I, but also those optionally substituted, can be achieved with variety of reducing agents and reaction conditions suitable for reduction of ester to alcohol group. It is preferred that the process is performed using complex metal hydride such as and preferably seleceted from lithium aluminum hydride and sodium borohydride, but also calcium aluminum hydride or lithium borohydride with or without the presence of an additive for enhancement of reactivity such as LiBr, CoCl₂, ZnCl₂, AlCl₃, in an organic solvent for from 15 min to 24 h at the temperature in the range of -10 °C to 150 °C. In the preferred embodiment the reaction is performed with lithium aluminum hydride in the organic solvent preferably in an ether or aromatic hydrocarbon, such as tetrahydrofuran, ethyl ether, diisopropylether, metyl t-butylether, monoglyme, diglyme, dioxane, benzene, toluene, and the mixture thereof with the reaction time between 30 min and 8 h, even more preferably, in tetrahydrofuran solution in the temperature range between -5 °C to 30 °C. In an alternative embodiment, the optionally substituted compound of formula I is treated with sodium borohydride in an organic solvent as above or selected from the group of alcohols, polyols or glymes with or without an additive for enhancement of reactivity, preferably in 2 h to 8 h of reaction time and in the temperature ranges between 20 °C to 100 °C. After suitable workup and purification the 2-hydroxypheylpropanol derivative (compound of formula **II**) is obtained and used in the next step.

In the proceeding step the compound of formula **II** having two hydroxyl moieties is transformed by treating with a reagent presenting at the same time activation of the alcohol moiety (hydroxyl group on the propyl chain) forming easily substitutable group (functioning as an activating group) on propyl chain and protection of the phenol group (functioning as a protecting group). The preferred reaction is esterifying. The suitable reagent is preferably an acid or its derivative and can be selected from the group of acids, acid halogenides or acid anhydrides yielding O-substituents, such as alkylcarboxy, arylcarboxy, sulphonyloxy, phosphoryloxy groups, preferably sulfonyloxy groups, preferably unsubstituted and substituted (C₁-C₆)-alkansulfonyloxy, 10-camphorsulfonyloxy and arene-sulfonyloxy, yet more preferably trifluoromethanesulfonyloxy and C₂-C₄ sulfonyloxy groups, most preferably benzenesulfonyloxy or ethanesulfonyloxy groups. In the preferred embodiment, 2-hydroxypheylpropanol derivative (compound of formula **II**) is reacted with at least two equivalents of ethanesulfonyl chloride or benzenesulfonyl chloride or anhydride in the presence of at least two equivalents of organic base, preferably tertiary amine or pyridine derivative, preferably triethylamine. The reaction mixture is preferably stirred until completion of the reaction. The choice of the solvent is not critical, and suitable solvents include, but are not limited to chlorinated solvents such as methylene chloride, chloroform or toluene or in some case even aqueous solvents. The reaction is usually performed from about -20 °C to 40 °C, preferably -5 °C to 30 °C, more preferably -20 °C to 10°C. The reaction temperature may be also elevated in order to increase the reaction rate.

Compounds of Formula **III** may be upon isolation and purification used in the next step. Alternatively compounds of Formula **III** can be used in next step without purification i.e. a single pot synthesis from compounds of Formula **II** to **V** or from **III** to **V** is envisaged. In case those and/or subsequent intermediates are not isolated and purified, it is advantageous to make and use less reactive compounds, such as benzenesulfonyloxy or ethanesulfonyloxy or propanesulfonyloxy or butanesulfonyloxy, or carboxyl or phosphoryl that is those where Y in the Formula III represents: R"- SO₂- where R" is selected from C₂-C₄ alkyl, preferably C₂H₅, or C₄H₉; completely or partially fluorinated C₁-C₄ alkyl, preferably CF₃ or C₄F₉ or CF₃-CH₂; halogen, preferably F; NMe₃(CH₂); or Y represents R'-C₆H₄-SO₂; where R' is H, or Br, Cl, or Y is R"'-CO, where R'" is an alkyl.

The group attached to propyl chain of the compound of Formula **III** can be further selectively substituted with an amine of formula NHR₁R₂ where R₁ and R₂ can be same or different selected from H or optionally substituted C₁-C₄ alkyl; preferably diisopropylamine giving compound of Formula **V**. However if the amine is stericaly hindered such as diisopropylamine, it shows weak nucleophilic character for the substitution of activated ester groups. Thus, the reaction rate can be increased with use of aprotic polar solvents, elevated temperature and pressure. It is preferred that the reaction between compound of Formula **III** and diisopropylamine is performed in a polar aprotic solvent such as acetonitrile, DMA, DMF, THF, DMSO, 1-methylpyrrolidinone preferably acetonitrile. Alternatively the reaction can be performed in an organic solvent, which does not mix with water such as chlorinated solvents or aromatic solvents optionally using phase transfer catalysis. The reaction may be performed at temperatures above 50°C, preferably at above 70 °C more preferably at about 80 °C for up to 2 weeks, preferably for 4-8 days at elevated pressure, preferably in a pressure bottle (avtoclave) above normal pressure, preferably below 20 atm most preferably at about 3 atm.

However if the group attached to propyl chain of the compound of Formula **III** is first converted into halo derivative, yielding compound of Formula **IV**, where halo is iodo and only in subsequent reaction step into amine derivative, the reaction times of both reactions are significantly shorter (i.e up to a day, preferably up to 6 hours) compared to those as described above. Alternatively steps from compound of Fomrual **III** via compound of Formula **IV** to compound of formula **V** may be performed as a combined step i.e. in a single pot synthesis. Also in that case overall reaction time is significantly shorter.

Thus it is preferred that in the next step the compound of Formula **III** is reacted with a source of halogen, preferably sodium iodide or bromide, preferably under pressure, preferably in a suitable solvent such as be acetonitrile, DMF DMA. In that reaction the O-substituent of the propyl chain is substituted with a halogen. The reaction may be performed at temperatures above 50°C, but preferably bellow 200 °C, preferably at above 70 °C, more preferably above 80 °C, most preferably at temperatures at about 115 °C; for a period from few minutes up to about half a day, preferably from 2 to 6 hours. Formed compound of Formula **IV**, where Y is as above can be used in subsequent reaction steps without extensive purification.

Halo substituent on the compound of Formula **IV** can now be further selectively substituted with an amine of formula NHR₁R₂ where R₁ and R₂ can be same or different selected from H or optionally substituted C₁-C₄ alkyl; preferably diisopropylamine givng compound of Formula **V**. It is preferred that the reaction between compound of Formula **IV** and amine is performed in a polar aprotic solvent such as acetonitrile, DMA, DMF, THF, DMSO, 1-methylpyrrolidinone preferably acetonitrile or alternatively the reaction can be performed in an organic solvent, which does not mix with water as described above. The reaction may be performed at temperatures above 50°C, but preferably bellow 200 °C, preferably at above 70 °C, more preferably above 80 °C, most preferably at about 115° for up to few hours, preferably for 2-6 hours days at elevated pressure, preferably in a pressure bottle (avtoclave) above normal pressure, preferably below 20 atm most preferably at about 3 atm. The obtained compound of formula **V** can be generally (and preferably in case of a tertiary amine) purified by acido - basic extraction.

In case the reaction steps from compound of Formula **III** to Formula **V** are combined by performing reaction with amine in presence of sodium iodide, the molar amount of sodium iodide may be (and preferably are) lower than molar amount of starting compound of Formula **III**. The reaction conditions may be same as when aminating the compound of Formula **IV**.

Although more reactive esters such as toluene sulfonyloxy or methylsulfonyloxy provide for quicker reaction times, especialy in case when performing the reaction in absence of sodium iodide, the use of less reactive esters such as benzenesulfonyloxy or ethylsulfonyloxy is advantageous because less and lower amounts of side products are formed.

Cleavage of the O-substitutent such as alkylcarbonyl, sulphonyl, phosphonyl or phosphoryl ester group from the optionally substituted compound of formula **V** or its analog yields optionally substituted title compound of formula **VI.** In the preferred embodiment this compound is N,N-diisopropyl-3-(2-hydroxy-5-methylphenyl)-3-phenylpropylamine Generally, this cleavage can be achieved by hydrolysis by any method known in the art, for example using a suitable base or acid. In the preferred embodiment the base can be metal salt of an alkoxide or metal hydroxide in a solution of at least one alcohol, ether, amide, ketone or polar aprotic solvent or water or a mixture thereof. Suitable bases include but are not limited to sodium or potassium hydroxide, methoxide, ethoxide, propoxide, isopropoxide, t-butoxide and t-pentanoxide, with tertiary alkoxide being preferred. Suitable solvents include but are not limited to alcohols as methanol, ethanol, n-propanol, isopropanol, t-butanol or t-pentanol; aprotic solvents as tetrahydrofurane, dioxane, acetonitrile, dimetylformamide, dimethylacetamide or water or a mixture thereof. The use of the same alkyl group in an alkoxide and solvent alcohol is preferred to prevent exchange of the groups, for example, potassium t-butoxide in t-butanol. Preferably, the reaction is carried out in a solution of t-butoxide in t-butanol with a few equivalents of water added. It is performed at the temperature from room temperature up to the boiling point of the solvent, preferably from about 40 °C to about 100 °C, with about 50 °C to about 70 °C being optimum, the maximum temperature being determined by the boiling point of the used solvent. The reaction mixture is maintained at the desired temperature until the reaction is substantially complete, usually 1 to 12 hours, and then it is cooled to room temperature.

For the isolation of the product the following workup may be used: water is added to the reaction mixture and it is further stirred for some time. The product is extracted with a (with water) non-miscible organic solvent. The organic fraction is washed with water, dried with a drying agent and evaporated under vacuum to form N,N-diisopropyl-3-(2-hydroxy-5-methylphenyl)-3-phenylpropylamine (compound of formula V) which can be further treated with (+)-L-tartaric acid in order to achieve resolution of the enantiomers. The resolution of enatiomers will however mean to include also any other usual method. The following examples are offered to illustrate aspects of the present invention, and are not intended to limit or define the present invention in any manner.

### Preparation of 3-(2-hydroxy-5-methylphenyl)-3-phenylpropanol (compound of formula II)

A solution of 7-methyl-4-phenyl-3,4-dihydrocoumarin (compound of formula I) (9.52 g, 40.0 mmol) in dry tetrahydrofuran (50 ml) is added drop-wise to the cooled (0 °C) suspension of lithium aluminum hydride (3.04 g, 80 mmol) in 100 ml of dry tetrahydrofuran. The reaction mixture is stirred for 1 hour at room temperature under inert atmosphere and then the reaction is quenched by careful addition of 50 ml of a mixture tetrahydrofuran and water (1:1) followed by acidification with 1 mol/l solution of hydrochloric acid (150 ml). The product is extracted with ethyl acetate. The combined organic phases are washed with water and brine, and dried over anhydrous magnesium sulphate. After removal of the solvents the compound of formula II is obtained in 97 % yield (9.68 g). It is recrystallized from diisopropylether yielding the product as white powder; mp = 112 - 115. ¹H NMR (300MHz, DMSO-*d*₆): δ [ppm] = 2.08 - 2.17 (5H, m, CH₃, CH₂), 3.29 - 3.31 (2H, m, CH₂), 4.38, 4.42 (2H, 2 x t, CH, OH), 6.63 (1 H, d, J = 8.0 Hz, 1 H-Ar), 6.76 (1 H, dd, J = 8.3, 1.7 Hz, 1 H-Ar), 7.00 (1 H, d, J = 1.7Hz, 1 H-Ar), 7.08 - 7.13 (2H, m, 1 H-Ph), 7.20 - 7.28 (3H, m, 4H-Ph), 9.04 (1 H, s, OH)
¹³C NMR (300MHz, DMSO-*d*₆): δ [ppm] 20.5, 37.6, 39.1, 59.3, 115.0, 125.6, 127.0, 127.3, 127.9, 128.0, 130.9, 145.3, 152.4.
ESI mass spectrum: 243 [M + H⁺]

### Preparation of 3-(2-hydroxy-5-methylphenyl)-3-phenylpropanol (compound of formula II)

Sodium borohydride (0.76 g, 20 mmol) is added in small portions to the cooled solution of 7-methyl-4-phenyl-3,4-dihydrocoumarin (compound of formula I) (0.48 g, 2 mmol) in 20 ml of methanol. The reaction mixture is stirred for 1 h at 0 °C and 4 h at room temperature. The reaction mixture is poured into 1 mol/l solution of hydrochloric acid (120 ml) and extracted with ethyl acetate (3 x 70 ml). The combined organic fractions are dried over anhydrous magnesium sulphate and the solvent evaporated to give the product in 70 % yield (0.49 g).

### Preparation of 3-(2-(p-toluenesulphonyloxy)-5-methylphenyl)-3-phenylpropyl-p-toluenesulphonate (compound of formula IIIa)

3-(2-hydroxy-5-methylphenyl)-3-phenylpropanol (1.15 g, 5.0 mmol) is suspended in 20 ml of dichloromethane, 4.20 ml of triethylamine (30 mmol) are added and the mixture is cooled to 0°C. After the addition of p-toluenesulfonylchloride (3.81 g, 20 mmol) the reaction mixture is stirred for 2 h at 0 °C, left to warm up slowly to the room temperature and stirred for additional 2 h. The reaction mixture is washed with ice-cold water (100 ml), cold 2 mol/l solution of hydrochloric acid (2 x 50 ml) and brine (50 ml). The organic phase is dried over anhydrous magnesium sulphate and the solvent evaporated to give oily residue. After purification with column chromatography using a mixture of hexane : ether = 2 : 1 as an eluent the product is obtained in 85 % yield (2.20 g).
¹H NMR (300MHz, CDCl₃): δ [ppm] = 2.12 - 2.32 (5H, m, CH₃, CH₂), 2.45, 2.47 (6H, 2 x s, 2 x CH₃), 3.79 - 3.98 (2H, m, 2 x CH), 4.20 (1 H, dd, J = 9.2, 6.7 Hz, CH), 6.91 - 7.00 (5H, m, Ph), 7.14 - 7.20 (3H, m, Ar), 7.31, 7.35 (4H, 2 x d, J = 8.6 Hz, 4H-Ts), 7.73, 7.77 (4H, 2 x d, J = 8.2 Hz, 4H-Ts).
¹³C NMR (300MHz, CDCl₃): δ [ppm] = 21.0, 21.5, 21.6, 33.7, 39.4, 68.3, 121.7, 126.5, 127.8, 128.1, 128.3, 128.4, 128.6, 129.7, 129.9, 132.7, 132.8, 136.0, 136.9, 141.3, 144.6, 145.2, 145.4.
ESI mass spectrum: 551 [M + H⁺]

### Preparation of 3-(2-methanesulphonyloxy-5-methylphenyl)-3-phenylpropyl-p-methanesulphonate (compound of formula IIIb)

3-(2-hydroxy-5-methylphenyl)-3-phenylpropanol (1.15 g, 4.8 mmol) is suspended in 20 ml of dichloromethane and 4.2 ml triethylamine (30 mmol) are added. The solution is cooled to -5 °C and methanesulfonyl chloride (1.55 ml, 20 mmol) is added drop-wise. The reaction mixture is stirred for 2 h at 0 °C and washed with ice-cold water (100 ml), cold 2 mol/l solution of hydrochloric acid (2 x 50 ml) and brine (50 ml). The organic phase is dried over anhydrous magnesium sulphate and solvent evaporated to give yellowish oily residue. After purification with column chromatography using dichloromethane as an eluent the product is obtained in 82 % yield (1.56 g).
¹H NMR (300MHz, CDCl₃): δ [ppm] = 2.34 (3H, s, CH₃), 2.48 (2H, dd, J = 14.4, 6.4 Hz, CH₂), 2.95, 3.01 (6H, 2 x s, 2 x CH₃), 4.17-4.22 (2H, m, CH₂), 4.57 (1H, t, J = 7.94 Hz, CH), 7.06 (H, dd, J = 8.3, 2.3 Hz, H-Ar), 7.13 (1H, d, J = 2.0 Hz, 1 H-Ar), 7.21 - 7.36 (6H, m, 5H-Ph, 1 H-Ar).
¹³C NMR (300MHz, CDCl₃): δ [ppm] = 21.1, 34.3, 37.2, 37.9, 39.8, 67.9, 121.6, 126.9, 128.0, 128.7, 128.8, 129.2, 135.7, 137.3, 141.8, 145.0
ESI mass spectrum: 399 [M + H⁺]

### Preparation of 3-(2-(benzenesulfonyloxy)-5-methylphenyl)-3-phenylpropyl-benzenesulfonate (compound of formula IIIc)

To a well stirred solution of 3-(2-hydroxy-5-methylphenyl)-3-phenylpropanol (4.84 g, 20 mmol) and 1,4-diaza-bicyclo[2.2.2]octane (DABCO, 5.61 g, 50 mmol) in 20 ml of dichloromethane at room temperature benzenesulfonyl chloride (5.6 ml, 44 mmol) is slowly added. The resulting mixture is refluxed for 2 hours. The reaction mixture is then cooled to room temperature and tert-butyl methyl ether (MTBE, 100 ml) is added. The mixture is washed with 1 mol/l solution of hydrochloric acid (2 x 50 ml), 5 % solution of sodium hydrogen carbonate (50 ml) and brine (50 ml). The organic phase is dried over anhydrous magnesium sulfate, filtrated and evaporated *in vacuo* to give the product as a colorless or slightly yellow oil in quantitative yield (10.4 g).
¹H NMR (300 MHz, CDCl₃): δ [ppm] = 2.12 - 2.36 (2H, m, CH₂), 2.25 (3H, s, CH₃), 3.80 - 3.89 (1 H, m, CH), 3.92 - 4.02 (1 H, m, CH), 4.21 (1 H, dd, J = 9.1, 6.7 Hz), 6.83 - 7.02 (5H, m, Ph), 7.09 - 7.23 (4H, m, 4H-Ar), 7.47 - 7.73 (6H, m, 6H-Ar), 7.81 - 7.99 (3H, m, 3H-Ar).

### Preparation of 3-(2-(ethanesulfonyloxy)-5-methylphenyl)-3-phenylpropyl-ethanesulfonate (compound of formula IIId)

3-(2-Hydroxy-5-methylphenyl)-3-phenylpropanol (4.84 g, 20 mmol) is suspended in 20 ml of dichloromethane. Triethylamine (7.0 ml, 50 mmol) is added and the resulting clear solution is cooled to 0 °C. After the slow addition of ethanesulfonyl chloride (4.2 ml, 44 mmol) the mixture is stirred for 15 minutes at 0 °C. *tert*-Butyl methyl ether (MTBE, 100 ml) is added. The mixture is washed with 1 mol/l solution of hydrochloric acid (2 × 50 ml), 5 % solution of sodium hydrogen carbonate (50 ml) and brine (50 ml). The organic phase is dried over anhydrous magnesium sulfate, filtrated and evaporated *in vacuo* to give the product as a brown oil in quantitative yield (8.5 g).
¹H NMR (300 MHz, CDCl₃): δ [ppm] = 1.37 (3H, t, J = 7.5 Hz, CH₂C*H*₃), 1.53 (3H, t, J = 7.5 Hz, CH₂C*H*₃), 2.32 (3H, s, CH₃), 2.43 - 2.53 (2H, m, CH₂), 3.07 (2H, q, J = 7.5 Hz, C*H*₂CH₃), 3.28 (2H, dq, J = 7.5, 1.4 Hz, C*H*₂CH₃), 4.22 - 4.37 (2H, m, CH₂), 4.60 (1 H, t, J = 7.9 Hz, CH), 7.02 - 7.07 (1 H, m, 1 H-Ar), 7.09 - 7.13 (1 H, m, 1 H-Ar), 7.19 - 7.35 (6H, m, 1 H-Ar, Ph).

### Preparation of 2-(3-iodo-1-phenylpropyl)-4-methylphenyl-benzenesulfonate (compound of formula IVc)

A mixture of 3-(2-(benzenesulfonyloxy)-5-methylphenyl)-3-phenylpropyl-benzenesulfonate (5.22 g, 10 mmol) and sodium iodide (1.88 g, 12.5 mmol) in 20 ml of acetonitrile is heated in a pressure bottle at 115 °C for 6 hours. After cooling the mixture is evaporated *in vacuo.* The residue is dissolved with vigorous stirring in 50 ml MTBE and 50 ml 1 mol/l solution of sodium hydroxide. The phases are separated and the aqueous phase is once more extracted with 50 ml of MTBE. Organic extracts are combined, washed with 50 ml of brine, dried over anhydrous magnesium sulfate, filtrated and evaporated *in vacuo* to give the product as light yellow solid (m.p. = 112.5 - 115.7 °C) in 92 % yield (4.5 g).
¹H NMR (300 MHz, CDCl₃): δ [ppm] = 2.28 - 2.40 (1 H, m, CH), 2.30 (3H, s, CH₃), 2.46 - 2.59 (1 H, m, CH), 2.84 - 2.94 (1 H, m, CH), 3.00 - 3.09 (1 H, m, CH), 4.21 (1 H, dd, J = 8.7, 6.7 Hz), 6.94 - 6.96 (2H, m, 2H-Ar), 7.03 - 7.06 (1 H, m, 1 H-Ar), 7.11 - 7.30 (5H, m, 5H-Ar), 7.52 - 7.61 (2H, m, 2H-Ar), 7.67 - 7.75 (1 H, m, 1 H-Ar), 7.91 - 7.93 (1 H, m, 1 H-Ar), 7.93 - 7.96 (1 H, m, 1 H-Ar).

### Preparation of 2-(3-iodo-1-phenylpropyl)-4-methylphenyl ethanesulfonate (compound of formula IVd)

A mixture of 3-(2-(ethanesulfonyloxy)-5-methylphenyl)-3-phenylpropyl-ethanesulfonate (8.5 g, 20 mmol) and sodium iodide (3.75 g, 25 mmol) in 40 ml of acetonitrile is heated in a pressure bottle at 115 °C for 2 hours. After cooling the mixture is evaporated *in vacuo.* The residue is dissolved with vigorous stirring in 50 ml MTBE and 50 ml of 1 mol/l solution of sodium hydroxide. The phases are separated and the aqueous phase is once more extracted with 50 ml of MTBE. Organic extracts are combined, washed with 50 ml of brine, dried over anhydrous magnesium sulfate, filtrated and evaporated *in vacuo* to give the product as brown oil in 99 % yield (8.8 g).
¹H NMR (300 MHz, CDCl₃): δ [ppm] = 1.53 (3H, t, J = 7.5 Hz, CH₂C*H*₃), 2.33 (3H, s, CH₃), 2.48 - 2.61 (2H, m, CH₂), 3.02 - 3.34 (4H, m, 2×CH₂), 4.53 (1 H, t, J = 7.6 Hz, CH), 7.02 - 7.07 (1 H, m, 1 H-Ar), 7.11 - 7.13 (1 H, m, 1 H-Ar), 7.19 - 7.35 (6H, m, 1 H-Ar, Ph).

### Preparation of N,N-diisopropyl-3-(2-(p-toluenesulphonyloxy)-5-methylphenyl)-3-phenylpropyl amine (compound of formula Va)

A mixture of 3-(2-(p-toluenesulphonyloxy)-5-methylphenyl)-3-phenylpropyl-p-toluenesulphonate (2.06 g, 3.74 mmol) and diisopropylamine (5.2 ml, 37.4 mmol) in 10 ml of acetonitrile is heated in a pressure bottle at 80 °C for 4 - 6 days. After cooling the reaction mixture the volatile components are evaporated and the residue is treated with 50 ml of 2 mol/l solution of sodium hydroxide and extracted with ether (3 x 50 ml).

The combined extracts are washed with water and extracted with 2 mol/l solution of hydrochloric acid (3 x 50 ml). To combined extracts 30 % aqueous sodium hydroxide solution is added until pH is adjusted to 13 - 14. The product is extracted with ether (3 x 50 ml), the combined extracts are washed with brine (70 ml) and dried over anhydrous magnesium sulphate. The solvent is evaporated and the product is obtained as an oily residue in 73 % yield (1.31 g).
¹H NMR (300MHz, CDCl₃): δ [ppm] = 0.93 (12H, d, J = 6.3 Hz, 4 x CH₃), 2.00 - 2.09 (2H, m, 2 x CH), 2.24 - 2.27 (5H, m, CH₃, CH₂), 2.45 (3H, s, CH₃), 2.93 - 2.99 (2H, m, CH₂), 4.16 (1H, t, J = 7.5 Hz, CH), 6.89 (2H, s, 2H-Ar), 7.10 - 7.27 (6H, m, 5H-Ph, 1 H-Ar), 7.32 (2H, d, J = 8.7 Hz, Ts), 7.78 (2H, d, J = 8.4 Hz, Ts)
¹³C NMR (300MHz, CDCl₃): δ [ppm] = 20.3, 20.5, 20.9, 21.5, 37.2, 41.6, 43.7, 48.8, 121.2, 126.0, 127.5, 128.0, 128.1, 128.2, 128.9, 129.6, 133.3, 136.5, 137.6, 143.2, 145.0, 145.5.
ESI mass spectrum: 480 [M + H⁺]

### Preparation of N,N-diisopropyl-3-2-(methanesulphonyloxy)-5-methylphenyl-3-phenylpropyl amine (compound of formula Vb)

A mixture of 3-(2-methanesulphonyloxy-5-methylphenyl)-3-phenylpropyl-p-methanesulphonate (1.66 g, 4.2 mmol) and diisopropylamine (5.9 ml) in 11 ml of . acetonitrile is heated in a pressure bottle at 80 °C for 4 - 6 days. Volatile components are evaporated and the residue is treated with 50 ml of 2 mol/l solution of sodium hydroxide and extracted with ether (3 x 50 ml). The combined extracts are washed with water (2 x 30 ml) and extracted with 2 mol/l solution of hydrochloric acid (3 x 50ml). The water extracts are combined and washed with ether (2 x 30 ml) and 30 % aqueous sodium hydroxide solution is added until pH is adjusted to 13 - 14. The product is extracted with ether; combined extracts are washed with brine and dried over anhydrous magnesium sulphate. The solvent is evaporated and the product is obtained as an oily residue in 67 % yield (1.12 g).
¹H NMR (300MHz, CDCl₃): δ [ppm] = 0.95 (12H, d, J = 6.4 Hz, 4 x CH₃), 2.14 - 2.18 (2H, m, 2 x CH), 2.35 - 2.40 (5H, m, CH₃, CH₂), 2.79 (3H, s, CH₃), 2.95 - 3.03 (2H, m, CH₂), 4.34 (1H, t, J = 7.6 Hz, CH), 7.03 (1 H, dd, J = 8.2, 2.0 Hz, 1 H-Ar), 7.16 - 7.29 (7H, m, 5H-Ph, 2H-Ar).
¹³C NMR (300 MHz, CDCl₃): δ [ppm] = 20.3, 20.5, 20.9, 37.0, 37.2, 41.7, 43.5, 48.6, 120.7, 126.1, 127.8, 128.0, 128.3, 129.2, 136.7, 136.9, 143.7, 145.4.
ESI mass spectrum: 404 [M + H⁺]

### Preparation of N,N-diisopropyl-3-(2-(benzenesulfonyloxy)-5-methylphenyl)-3-phenylpropyl amine (compound of formula Vc)

A mixture of 3-(2-(benzenesulfonyloxy)-5-methylphenyl)-3-phenylpropyl-benzenesulfonate (5.22 g, 10 mmol), diisopropylamine (8.6 ml, 60 mmol) and sodium iodide (0.75 g, 5 mmol) in 24 ml of acetonitrile is heated in a pressure bottle at 115 °C for 6 hours. After cooling the mixture is evaporated *in vacuo.* The residue is dissolved with vigorous stirring in 25 ml MTBE and 25 ml 2 mol/l solution of sodium hydroxide. The phases are separated and the aqueous phase is once more extracted with 25 ml of MTBE. Organic extracts are combined, washed with 25 ml of water and extracted with 2 mol/l solution of hydrochloric acid (2 × 25 ml). The MTBE phases are discarded. The acidic phases are combined and basified with 4 mol/l solution of sodium hydroxide to pH = 13. The mixture is extracted with MTBE (2 × 25 ml). Organic phases are combined, washed with 25 ml of brine, dried over anhydrous magnesium sulfate, filtrated and evaporated *in vacuo* to give the product as brown oil in 75 % yield (3.5 g).
¹H NMR (300 MHz, CDCl₃, CD₃OD): δ [ppm] = 0.78 (12H, d, J = 6.4 Hz, 4 × CH₃), 1.82 - 2.03 (2H, m, CH₂), 2.07 (3H, s, CH₃), 2.07 - 2.19 (2H, m, CH₂), 2.76 - 2.88 (2H, m, 2 × CH), 3.92 (1 H, t, J = 7.6 Hz), 6.57 - 6.61 (1 H, m, 1 H-Ar), 6.68 - 6.72 (1H, m, 1 H-Ar), 6.87 - 7.09 (6H, m, 6H-Ar), 7.33 - 7.42 (2H, m, 2H-Ar), 7.49 - 7.57 (1 H, m, 1 H-Ar), 7.66 - 7.72 (2H, m, 2H-Ar).

### Preparation of N,N-diisopropyl-3-(2-(benzenesulfonyloxy)-5-methylphenyl)-3-phenylpropyl amine (compound of formula Vc)

A mixture of 2-(3-iodo-1-phenylpropyl)-4-methylphenyl benzenesulfonate (4.38 g, 8.9 mmol), diisopropylamine (4.3 ml, 30 mmol) in 12 ml of acetonitrile is heated in a pressure bottle at 115 °C for 6 hours. After cooling the mixture is evaporated *in vacuo.* The residue is dissolved with vigorous stirring in 25 ml MTBE and 25 ml 2 mol/l solution of sodium hydroxide. The phases are separated and the aqueous phase is once more extracted with 25 ml of MTBE. Organic extracts are combined, washed with 25 ml of water and extracted with 2 mol/l solution of hydrochloric acid (2 × 25 ml). The MTBE phases are discarded. The acidic phases are combined and basified with 4 mol/l solution of sodium hydroxide to pH = 13. The mixture is extracted with MTBE (2 × 25 ml). Organic phases are combined, washed with 25 ml of brine, dried over anhydrous magnesium sulfate, filtrated and evaporated *in vacuo* to give the product as brown oil in 77 % yield (3.2 g).

### Preparation of N,N-diisopropyl-3-(2-(ethanesulfonyloxy)-5-methylphenyl)-3-phenylpropyl amine (compound of formula Vd)

A mixture of 3-(2-(ethanesulfonyloxy)-5-methylphenyl)-3-phenylpropyl-ethanesulfonate (8.53 g, 20 mmol), diisopropylamine (17 ml, 120 mmol) and sodium iodide (1.5 g, 10 mmol) in 48 ml of acetonitrile is heated in a pressure bottle at 115 °C for 6 hours. After cooling the mixture is evaporated *in vacuo.* The residue is dissolved with vigorous stirring in 50 ml MTBE and 50 ml 2 mol/l solution of sodium hydroxide. The phases are separated and the aqueous phase is once more extracted with 50 ml of MTBE. Organic extracts are combined, washed with 50 ml of water and extracted with 2 mol/l solution of hydrochloric acid (2 × 50 ml). The MTBE phases are discarded. The acidic phases are combined and basified with 4 mol/l solution of sodium hydroxide to pH = 13. The mixture is extracted with MTBE (2 × 50 ml). Organic phases are combined, washed with 50 ml of brine, dried over anhydrous magnesium sulfate, filtrated and evaporated *in vacuo* to give the product as brown oil in 91 % yield (7.6 g).
¹H NMR (300 MHz, CDCl₃): δ [ppm] = 0.95 (12H, d, J = 6.4 Hz, 4 × CH₃), 1.48 (3H, t, J = 7.4 Hz, CH₂C*H*₃), 2.09 - 2.22 (2H, m, CH₂), 2.33 (3H, s, CH₃), 2.34 - 2.43 (2H, m, CH₂), 2.92 - 3.06 (2H, m, 2 × CH), 3.07 - 3.24 (2H, m, CH₂) 4.38 (1 H, t, J = 7.6 Hz), 6.98 - 7.03 (1 H, m, 1 H-Ar), 7.14 - 7.23 (3H, m, 3H-Ar), 7.24-7.33 (4H, m, 4H-Ar), 6.87 - 7.09 (6H, m, 6H-Ar), 7.33 - 7.42 (2H, m, 2H-Ar), 7.49 - 7.57 (1 H, m, 1 H-Ar), 7.66 - 7.72 (2H, m, 2H-Ar).

### Preparation of N,N-diisopropyl-3-(2-(ethanesulfonyloxy)-5-methylphenyl)-3-phenylpropyl amine (compound of formula Vd)

A mixture of 2-(3-iodo-1-phenylpropyl)-4-methylphenyl ethanesulfonate (8,8 g, 19.8 mmol), diisopropylamine (8.6 ml, 60 mmol) in 24 ml of acetonitrile is heated in a pressure bottle at 115 °C for 6 hours. After cooling the mixture is evaporated *in vacuo.* The residue is dissolved with vigorous stirring in 50 ml MTBE and 50 ml 2 mol/l solution of sodium hydroxide. The phases are separated and the aqueous phase is once more extracted with 50 ml of MTBE. Organic extracts are combined, washed with 50 ml of water and extracted with 2 mol/l solution of hydrochloric acid (2 × 50 ml). The MTBE phases are discarded. The acidic phases are combined and basified with 4 mol/l solution of sodium hydroxide to pH = 13. The mixture is extracted with MTBE (2 × 50 ml). Organic phases are combined, washed with 50 ml of brine, dried over anhydrous magnesium sulfate, filtrated and evaporated *in vacuo* to give the product as brown oil in 90 % yield (7.5 g).

### Preparation of N,N-diisopropyl-3-(2-hydroxy-5-methylphenyl)-3-phenylpropyl amine (compound of formula VI)

To N,N-diisopropyl-3-(2-(p-toluenesulphonyloxy)-5-methylphenyl)-3-phenylpropyl amine (compound of formula IVa) (1.05 g, 1.91 mmol) 27 ml of deoxygenated 1 mol/l solution of potassium t-butoxide in t-butanol and 0.65 ml of deoxygenated deionised water are added. The reaction is performed under flow of nitrogen. The reaction mixture is heated to 65 °C and stirred at this temperature for 3.5 h. After cooling to room temperature 150 ml of deionised water is added to the reaction mixture and stirred for 1 h. The product is extracted with ether (3 x 100 ml). The combined extracts are washed with water (100 ml) dried over anhydrous magnesium sulphate and the solvent evaporated giving the product as oily residue in 95% yield (0.59 g).

### Enantiomeric resolution of N,N-diisopropyl-3-(2-hydroxy-5-methylphenyl)-3-phenylpropyl amine (compound of formula VI)

N,N-diisopropyl-3-(2-hydroxy-5-methylphenyl)-3-phenylpropylamine (compound of formula V) (0.49 g, 1.5 mmol) is dissolved in 5 ml of hot 96 % ethanol and added to the hot solution (+)-L-tartaric acid (0.23 g, 1.5 mmol) in 5 ml 96 % ethanol. The mixture is heated to the boiling point and filtrated. The filtrate is cooled to 5 °C and kept at this temperature for 24 h. Formed white precipitate is filtered off and washed with ethanol. The precipitate is recrystallized from ethanol to yield (+)-N,N-diisopropyl-3-(2-hydroxy-5-methylphenyl)-3-phenylpropylamine hydrogen tartrate in 36 % yield (0.26 g).

## Claims

1. A process for preparing N,N-diisopropyl-3-(2-hydroxy-5-methylphenyl)-3-phenylpropylamine or a salt thereof **characterized in that** the compound of following formula is used: 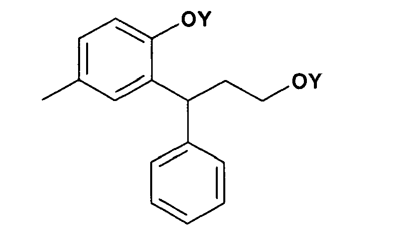 where Y can be: R"- SO₂- where R" is selected from C₂-C₄ alkyl, preferably C₂H₅, or C₄H₉; completely or partially fluorinated C₁-C₄ alkyl, preferably CF₃ or C₄F₉ or CF₃-CH₂; halogen, preferably F; NMe₃(CH₂); or Y represents R'-C₆H₄-SO₂; where R' is H, or halogen, or Y is R"'-CO, where R"' is selected from C₁-C₃ alkyl.

2. A process for preparing N,N-diisopropyl-3-(2-hydroxy-5-methylphenyl)-3-phenylpropylamine or a salt thereof **characterized in that** 3-(2-hydroxy-5-methylphenyl)-3-phenylpropanol is transformed into compound of formula: 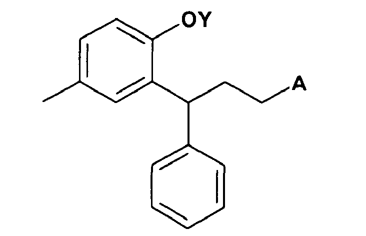 where Y can be: H,
or Y can be: COR where
R is selected from C₁-C₃ alkyl;
or Y can be: P(OR)₂ where
R is selected from C₁-C₃ alkyl;
or Y can be: PX(Z)₂ where
X is selected from O, N-SO₂-C₆H₄-Me NPh; and Z is selected from
OPh, NMe₂;
or Y can be: Ar-SO₂ where Ar is R'-C₆H₄ where
R' is selected from H, halogen, NO₂;
or Y can be: R"- SO₂- where
R" is selected from C₂-C₄ alkyl, fluorinated C₁-C₄ alkyl, halogen, NMe₃(CH₂)⁺;
and A is OY,
further **characterized in that** A is subsequently transformed into I or Br; and into N(i-Pr)₂, and if desired the obtained compound is converted to salt.

3. A process comprising the following steps:
a) reductive lactone ring opening of 3,4-dihydro-6-methyl-4-phenyl-2-benzopyran-2-one to yield 3-(2-hydroxy-5-methylphenyl)-3-phenylpropanol;
b) transforming both hydroxy groups of the compound obtained in previous step with the same reagent to form di-O-substituted derivative, wherein the hydroxy groups of 3-(2-hydroxy-5-methylphenyl)-3-phenylpropanol are esterified with an acid or a halide or anhydride of an acid selected from group consisting of: benzensulfonic acid, 4-bromobenzenesulfonic acid, 4-nitrobenzenesulfonic acid, ethanesulfonic acid, propanesulfonic acid, butansulfonic acid, trifluoromethanesulfonic acid, 2,2,2-trifluoroethanesulfonic acid, nonafluorobutanesulfonic acid, and fluorosulfonic acid;
c) substituting the O-substitutent on the propyl chain of the compound obtained in previous step with a halogen;
d) substituting the halogen on the propyl chain of the compound obtained in previous step with an amine;
e) hydrolysing the compound obtained in previous step to transform the remaining O-substitutent into hydroxy substituent; and
f) optionally optically resolving the mixture of enantiomers obtained in any of the previous steps.

4. The process according to the previous claim where steps c) and d) are performed as a combined step.

5. The process according to any of the previous two claims where steps b) to d) are performed in a single pot.

6. The process according to claim 3 in which esters are prepared by reacting halides or anhydrides of acids selected from the group consisting of: benzensulfonic acid or ethanesulfonic acid with 3-(2-hydroxy-5-methylphenyl)-3-phenylpropanol in the presence of an organic base.

7. The process according to previous claim wherein an organic base is pyridine, substituted pyridine or tertiary amine.

8. The process according to any of the claims 2 to 7 **characterized in that** substituting the substituent on the propyl chain with an amine performed in presence of a halide.

9. A process for preparing N,N-diisopropyl-3-(2-hydroxy-5-methylphenyl)-3-phenylpropylamine characterized that in one of the steps the compound of formula: 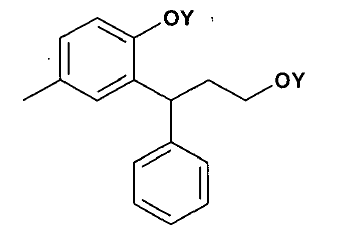 where Y can be: COR where
R is selected from C₁-C₃ alkyl;
or Y can be: P(OR)₂ where
R is selected from C₁-C₃ alkyl;
or Y can be: PX(Z)₂ where
X is selected from O, N-SO₂-C₆H₄-Me, NPh; and Z is selected from OPh, NMe₂;
or Y can be: Ar-SO₂ where Ar is R'-C₆H₄ where
R' is selected from H, halogen, NO₂;
or Y can be: R"- SO₂- where
R" is selected from C₂-C₄ alkyl, fluorinated C₁-C₄ alkyl, halogen, NMe₃(CH₂)⁺,
is reacted with an amine in presence of a halide.

10. A process according to any of the previous two claims **characterized in that** the halide is sodium iodide.

11. The process according to any one of the claims 2 to 10 wherein substituting the substituent on the propyl chain with an amine is performed in a polar aprotic solvent, preferably acetonitrile.

12. The process according to any of the claims 2 to 11 **characterized in that** the reaction with amine performed at temperature above 70 °C.

13. The process according to any of the claims 2 to 12 **characterized in that** the reaction with amine is performed at pressure of above 1 atm.

14. The process according to any of the preceding claims, wherein N,N-diisopropyl-3-(2-hydroxy-5-methylphenyl)-3-phenylpropylamine is further converted into (+)-N,N-diisopropyl-3-(2-hydroxy-5-methylphenyl)-3-phenylpropylamine hydrogen tartrate, and optionally incorporated into pharmaceutical composition.

15. A compound of formula: 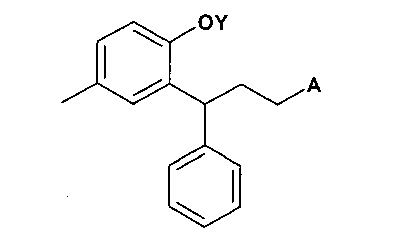 where Y can be: H,
or Y can be: COR where
R is selected from C₁-C₃ alkyl or Y can be: P(OR)₂ where
R is selected from C₁-C₃ alkyl;
or Y can be: PX(Z)₂ where
X is selected from O, N-SO₂-C₆H₄-Me, NPh; and Z is selected from OPh, NMe₂;
or Y can be: Ar-SO₂ where Ar is R'-C₆H₄ where
R' is selected from H, halogen, NO₂;
or Y can be: R"- SO₂- where
R" is selected from C₂-C₄ alkyl,halogen, NMe₃(CH₂)⁺ ;
and A is I or Br.

16. Compound selected from: 3-(2-(benzenesulphonyloxy)-5-methylphenyl)-3-phenylpropyl-p-benzenesulphonate; 3-(2-Ethanesulphonyloxy-5-methylphenyl)-3-phenylpropyl-ethanesulphonate; N,N-Diisopropyl-3-(2-(benzenesulphonyloxy)-5-methylphenyl)-3-phenylpropyl amine, N,N-Diisopropyl-3-(2-ethanesulphonyloxy-5-methylphenyl)-3-phenylpropyl amine, 3-(2-(benzenesulphonyloxy)-5-methylphenyl)-3-phenylpropyl iodide, and 3-(2-ethanesulphonyloxy-5-methylphenyl)-3-phenylpropyl iodide.

17. 3-(2-(benzenesulphonyloxy)-5-methylphenyl)-3-phenylpropyl iodide, and 3-(2-ethanesulphonyloxy-5-methylphenyl)-3-phenylpropyl iodide.

18. Use of sodium iodide in the process of manufacturing N,N-diisopropyl-3-(2-hydroxy-5-methylphenyl)-3-phenylpropyl amine.

## Patentansprüche

1. Verfahren zur Herstellung von N,N-Diisopropyl-3-(2-hydroxy-5-methylphenyl)-3-phenylpropylamin oder einem Salz davon, **dadurch gekennzeichnet, dass** die Verbindung der folgenden Formel verwendet wird: 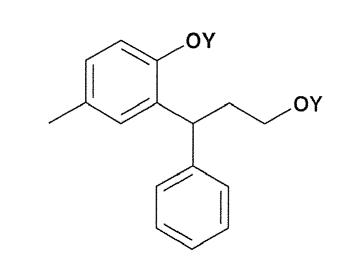 worin Y sein kann: R'-SO₂, wobei R" aus C₂-C₄-Alkyl, vorzugsweise C₂H₅ oder C₄H₉, ausgewählt ist; vollständig oder teilweise fluoriertes C₁-C₄-Alkyl, vorzugsweise CF₃ oder C₄F₉ oder CF₃-CH₂; Halogen, vorzugsweise F; NMe₃(CH₂) ; oder Y R'-C₆H₄-SO₂ darstellt;
wobei R'
H oder Halogen ist, oder
Y R"'-CO ist, wobei R"' aus C₁-C₃-Alkyl ausgewählt ist.

2. Verfahren zur Herstellung von N,N-Diisopropyl-3-(2-hydroxy-5-methylphenyl)-3-phenylpropylamin oder einem Salz davon, **dadurch gekennzeichnet, dass** 3-(2-Hydroxy-5-methylphenyl)-3-phenylpropanol in eine Verbindung der folgenden Formel umgewandelt wird: 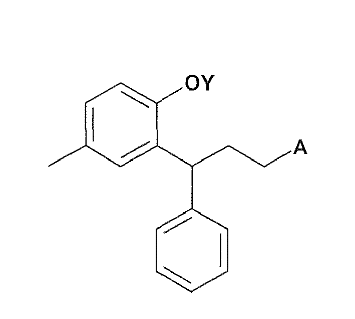 wobei Y sein kann: H,
oder Y sein kann: COR, wobei
R aus C₁-C₃-Alkyl ausgewählt ist;
oder Y sein kann: P(OR)₂, wobei
R aus C₁-C₃-Alkyl ausgewählt ist;
oder Y sein kann: PX(Z)₂, wobei
X aus O, N-SO₂-C₆H₄-Me, NPh ausgewählt ist; und Z aus OPh, NMe₂ ausgewählt ist;
oder Y sein kann: Ar-SO₂, wobei Ar R'-C₆H₄ ist, wobei
R' aus H, Halogen, NO₂ ausgewählt ist;
oder Y sein kann: R'-SO₂, wobei
R" aus C₂-C₄-Alkyl, fluorinertem C₁-C₄-Alkyl, Halogen, NMe₃(CH₂)⁺ ausgewählt ist;
und A OY ist,
weiterhin **gekennzeichnet dadurch, dass** A danach in I oder Br, und in N(i-Pr)₂ transformiert wird, und wenn gewünscht die erhaltene Verbindung in Salz umgewandelt wird.

3. Verfahren umfassend die folgenden Schritte:
a) Reduktive Lakton-Ringöffnung von 3,4-Dihydro-6-methyl-4-phenyl-2-benzopyran-2-on, um 3-(2-Hydroxy-5-methylphenyl)-3-phenylpropanol zu erhalten;
b) Umwandeln beider Hydroxylgruppen der im vorhergehenden Schritt erhaltenen Verbindung mit demselben Reagenz, um ein di-O-substituiertes Derivat zu bilden, wobei die Hydroxylgruppen von 3-(2-Hydroxy-5-methylphenyl)-3-phenylpropanol verestert werden mit einer Säure oder einem Halid oder Anhydrid einer Säure, ausgewählt aus der Gruppe bestehend aus: Benzolsulfonsäure, 4-Brombenzolsulfonsäure, 4-Nitrobenzolsulfonsäure, Ethansulfonsäure, Propansulfonsäure, Butansulfonsäure, Trifluormethansäure, 2,2,2-Trifluorethansulfonsäure, Nonafluorbutansulfonsäure und Fluorsulfonsäure;
c) Substituieren des O-Substituenten in der Propylkette der im vorhergehenden Schritt erhalten Verbindung mit einem Halogen;
d) Substituieren des Halogens in der Propylkette der im vorhergehenden Schritt erhaltenen Verbindung mit einem Amin;
e) Hydrolysieren der im vorhergehenden Schritt erhaltenen Verbindung, um den verbleibenden O-Substituenten in einen Hydroxy-Substituenten zu transformieren; und
f) wahlweise optisches Trennen der in einem der vorhergehenden Schritte erhaltenen Mischung von Enantiomeren.

4. Verfahren gemäß dem vorhergehenden Anspruch, wobei Schritte c) und d) als ein kombinierter Schritt durchgeführt werden.

5. Verfahren gemäß einem der vorhergehenden zwei Ansprüche, wobei Schritte b) bis d) in einem einzigen Gefäß durchgeführt werden.

6. Verfahren gemäß Anspruch 3, in welchem durch Reagieren von Haliden oder Anhydriden von Säuren, ausgewählt aus der Gruppe bestehend aus: Benzolsulfonsäure oder Ethansulfonsäure, mit 3-(2-Hydroxy-5-methylphenyl)-3-phenylpropanol in der Gegenwart einer organischen Base Ester hergestellt werden.

7. Verfahren gemäß dem vorhergehenden Anspruch, wobei eine organische Base Pyridin, substituiertes Pyridin oder tertiäres Amin ist.

8. Verfahren gemäß einem der Ansprüche 2 bis 7, **dadurch gekennzeichnet, dass** das Substituieren des Substituenten in der Propylkette mit einem Amin in Gegenwart eines Halids durchgeführt wird.

9. Verfahren zur Herstellung von N,N-Diisopropyl-3-(2-hydroxy-5-methylphenyl)-3-phenylpropylamin,
**dadurch gekennzeichnet, dass** in einem der Schritte die Verbindung der Formel: 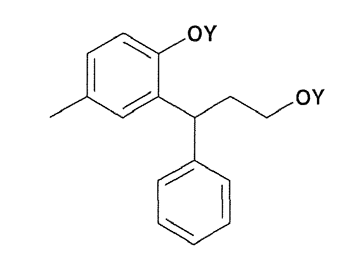 mit einem Amin in der Gegenwart eines Halids reagiert wird,
wobei Y sein kann: COR, wobei
R aus C₁-C₃-Alkyl ausgewählt ist;
oder Y sein kann: P(OR)₂, wobei
R aus C₁-C₃ Alkyl ausgewählt ist;
oder Y sein kann: PX(Z)₂, wobei
X aus O, N-SO₂-C₆H₄-Me, NPh ausgewählt ist; und Z aus OPh, NMe₂ ausgewählt ist;
oder Y sein kann: Ar-SO₂ wobei Ar R'-C₆H₄ ist, wobei
R' aus H, Halogen, NO₂ ausgewählt ist;
oder Y sein kann: R'-SO₂, wobei
R" aus C₂-C₄-Alkyl, fluoriertem C₁-C₄-Alkyl, Halogen, NMe₃(CH₂)⁺, ausgewählt ist.

10. Verfahren gemäß einem der vorhergehenden zwei Ansprüche, **dadurch gekennzeichnet, dass** das Halid Natriumiodid ist.

11. Verfahren gemäß einem der Ansprüche 2 bis 10, wobei das Substituieren des Substituenten in der Propylkette mit einem Amin in einem polaren aprotischen Lösungsmittel, vorzugsweise Acetonitril, durchgeführt wird.

12. Verfahren gemäß einem der Ansprüche 2 bis 11, **dadurch gekennzeichnet, dass** die Reaktion mit Amin bei einer Temperatur oberhalb von 70 °C durchgeführt wird.

13. Verfahren gemäß einem der Ansprüche 2 bis 12, **dadurch gekennzeichnet, dass** die Reaktion mit Amin bei einem Druck von über 1 atm durchgeführt wird.

14. Verfahren gemäß einem der vorhergehenden Ansprüche, wobei N,N-Diisopropyl-3-(2-hydroxy-5-methylphenyl)-3-phenylpropylamin
weiterhin in (+)-N,N-Diisopropyl-3-(2-hydroxy-5-methylphenyl)-3-phenylpropylamin-Hydrogentartrat umgewandelt wird, und optional in eine pharmazeutische Zusammensetzung eingebunden wird.

15. Verbindung der Formel: 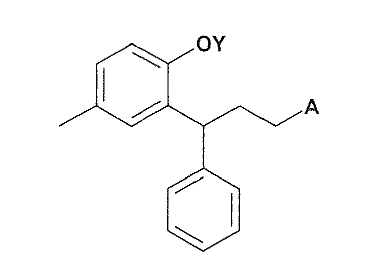 wobei Y sein kann: H,
oder Y sein kann: COR, wobei
R aus C₁-C₃-Alkyl ausgewählt ist
oder Y sein kann: P(OR)₂, wobei
R ausgewählt ist aus C₁-C₃-Alkyl;
oder Y sein kann: PX(Z)₂, wobei
X ausgewählt ist aus O, N-SO₂-C₆H₄-Me, NPh; und Z aus OPh, NMe₂ ausgewählt ist;
oder Y sein kann: Ar-SO₂, wobei Ar R'-C₆H₄ ist, wobei
R' aus H, Halogen, NO₂ ausgewählt ist;
oder Y sein kann: R"-SO₂-, wobei
R" aus C₂-C₄-Alkyl, Halogen, NMe₃(CH₂)⁺ ausgewählt ist;
und A I oder Br ist.

16. Verbindung ausgewählt aus 3-(2-(Benzolsulphonyloxy)-5-methylphenyl)-3-phenylpropyl-p-benzolsulfonat; 3-(2-Ethanesulfonyloxy-5-methylphenyl)-3-phenylpropyl-ethanesulfonat; N,N-Diisopropyl-3-(2-(benzolsulfonyloxy)-5-methylphenyl)-3-phenylpropylamin, N,N-Diisopropyl-3-(2-ethanesulfonyloxy-5-methylphenyl)-3-phenylpropylamin, 3-(2-(Benzolsulfonyloxy)-5-methylphenyl)-3-phenylpropyliodid, und 3-(2-Ethanesulfonyloxy-5-methylphenyl)-3-phenylpropyliodid.

17. 3-(2-(Benzolsulphonyloxy)-5-methylphenyl)-3-phenylpropyliodid, und 3-(2-Ethanesulfonyloxy-5-methylphenyl)-3-phenylpropyliodid.

18. Verwendung von Natriumiodid im Verfahren der Herstellung von N,N-Diisopropyl-3-(2-hydroxy-5-methylphenyl)-3-phenylpropylamin.

## Revendications

1. Procédé de préparation de N,N-diisopropyle-3-(2-hydroxy-5-méthylphényle)-3-phénylpropylamine
ou d'un sel de celle-ci, **caractérisé en ce que** le composé de formule suivante est utilisé : 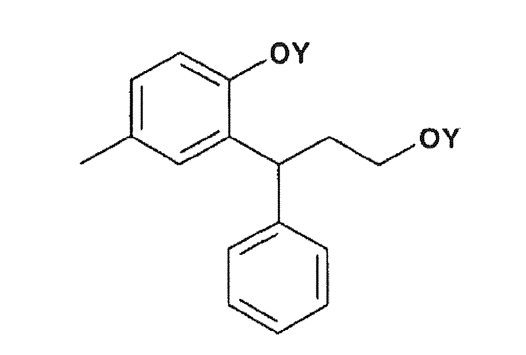 dans laquelle Y peut être : R"-SO₂- où R" est sélectionné parmi un groupe alkyle en C₂-C₄, de préférence C₂H₅ ou C₄H₉ ; un groupe alkyle en C₁-C₄ totalement ou partiellement fluoré, de préférence CF₃ ou C₄F₉ ou CF₃-CH₂ ; un atome d'halogène, de préférence F ; NMe₃(CH₂) ; ou Y représente R'-C₆H₄-SO₂
où R'
est H, ou un atome d'halogène, ou Y est R"'-CO, où R"' est sélectionné parmi un groupe alkyle en C₁-C₃.

2. Procédé de préparation de N,N-diisopropyle-3-(2-hydroxy-5-méthylphényle)-3-phénylpropylamine
ou d'un sel de celle-ci **caractérisé en ce que** le 3-(2-hydroxy-5-méthylphényle)-3-phénylpropanol est transformé en un composé de formule : 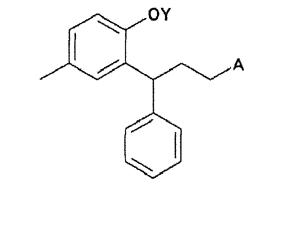 dans laquelle Y peut être : H,
ou Y peut être : COR où
R est sélectionné parmi un groupe alkyle en C₁-C₃ ; ou Y peut être : P(OR)₂ où
R est sélectionné parmi un groupe alkyle en C₁-C₃ ; ou Y peut être : PX(Z)₂ où
X est sélectionné parmi O, N-SO₂-C₆H₄-Me, NPh ; et Z est sélectionné parmi OPh, NMe₂ ;
ou Y peut être : Ar-SO₂ où Ar est R'-C₆H₄ où
R' est sélectionné parmi H, un atome d'halogène, NO₂ ;
ou Y peut être : R"-SO₂- où
R" est sélectionné parmi un groupe alkyle en C₂-C₄, un groupe alkyle en C₁-C₄ fluoré, un atome d'halogène, NMe₃(CH₂)⁺ ;
et A est OY,
en outre **caractérisé en ce que** A est ensuite transformé en I ou Br ; et en N(i-Pr)₂, et si souhaité, le composé obtenu est converti en un sel.

3. Procédé comprenant les étapes suivantes :
a) l'ouverture réductrice de cycle lactone du 3,4-dihydro-6-méthyl-4-phényl-2-benzopyran-2-one pour obtenir le 3-(2-hydroxy-5-méthylphényle)-3-phénylpropanol ;
b) la transformation des deux groupes hydroxy du composé obtenu dans l'étape précédente avec le même réactif pour former un dérivé di-O substitué, dans lequel les groupes hydroxy du 3-(2-hydroxy-5-méthylphényle)-3-phénylpropanol sont estérifiés avec un acide ou un halogénure ou un anhydride d'un acide sélectionné dans le groupe constitué par : l'acide benzènesulfonique, de l'acide 4-bromobenzènesulfonique, l'acide 4-nitrobenzènesulfonique, l'acide éthanesulfonique, l'acide propanesulfonique, l'acide butanesulfonique, l'acide trifluorométhanesulfonique, l'acide 2,2,2-trifluoroéthanesulfonique, l'acide nonafluorobutanesulfonique, et l'acide fluorosulfonique ;
c) la substitution du O-substituant sur la chaîne propyle du composé obtenu dans l'étape précédente avec un atome d'halogène ;
d) la substitution de l'atome d'halogène sur la chaîne propyle du composé obtenu à l'étape précédente avec une amine ;
e) l'hydrolyse du composé obtenu à l'étape précédente pour transformer le O-substituant restant en substituant hydroxy ; et
f) la résolution optique facultative du mélange d'énantiomères obtenu dans l'une quelconque des étapes précédentes.

4. Procédé selon la revendication précédente, dans lequel les étapes c) et d) sont réalisées sous la forme d'une étape combinée.

5. Procédé selon l'une quelconque des deux revendications précédentes, dans lequel les étapes b) à d) sont réalisées dans un récipient unique.

6. Procédé selon la revendication 3, dans lequel les esters sont préparés par réaction d'halogénures ou d'anhydrides d'acides sélectionnés dans le groupe constitué par : l'acide benzènesulfonique ou l'acide éthanesulfonique avec du 3-(2-hydroxy-5-méthylphényle)-3-phénylpropanol en présence d'une base organique.

7. Procédé selon la revendication précédente, dans lequel une base organique est la pyridine, la pyridine substituée ou une amine tertiaire.

8. Procédé selon l'une quelconque des revendications 2 à 7, **caractérisé en ce que** la substitution du substituant sur la chaîne propyle avec une amine est réalisée en présence d'un halogénure.

9. Procédé de préparation de N,N-diisopropyle-3-(2-hydroxy-5-méthylphényle)-3-phénylpropylamine
caractérisé en ce dans l'une des étapes, le composé de formule : 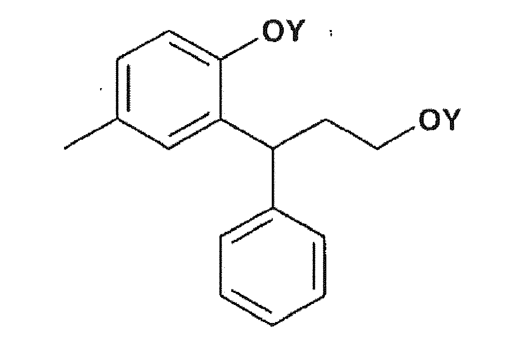 dans laquelle Y peut être : COR où
R est sélectionné parmi un groupe alkyle en C₁-C₃ ; ou Y peut être : P(OR)₂ où
R est sélectionné parmi un groupe alkyle en C₁-C₃ ; ou Y peut être : PX(Z)₂ où
X est sélectionné parmi O, N-SO₂-C₆H₄-Me, NPh ; et Z est sélectionné parmi OPh, NMe₂ ;
ou Y peut être : Ar-SO₂ où Ar est R'-C₆H₄ où
R' est sélectionné parmi H, un atome d'halogène, NO₂ ;
ou Y peut être : R"-SO₂- où
R" est sélectionné parmi un groupe alkyle en C₂-C₄, un groupe alkyle en C₁-C₄ fluoré, un atome d'halogène, NMe₃(CH₂)⁺,
est mis à réagir avec une amine en présence d'un halogénure.

10. Procédé selon l'une quelconque des deux revendications précédentes, **caractérisé en ce que** l'halogénure est l'iodure de sodium.

11. Procédé selon l'une quelconque des revendications 2 à 10, dans lequel la substitution du substituant sur la chaîne propyle avec une amine est réalisée dans un solvant aprotique polaire, de préférence l'acétonitrile.

12. Procédé selon l'une quelconque des revendications 2 à 11, **caractérisé en ce que** la réaction avec l'amine est réalisée à une température supérieure à 70°C.

13. Procédé selon l'une quelconque des revendications 2 à 12, **caractérisé en ce que** la réaction avec l'amine est réalisée à une pression supérieure à 1 atm.

14. Procédé selon l'une quelconque des revendications précédentes, dans lequel la N,N-diisopropyle-3-(2-hydroxy-5-méthylphényle)-3-phénylpropylamine est ensuite convertie en hydrogénotartrate de (+)-N,N-diisopropyle-3-(2-hydroxy-5-méthylphényle)-3-phénylpropylamine, et facultativement incorporée dans une composition pharmaceutique.

15. Composé de formule : 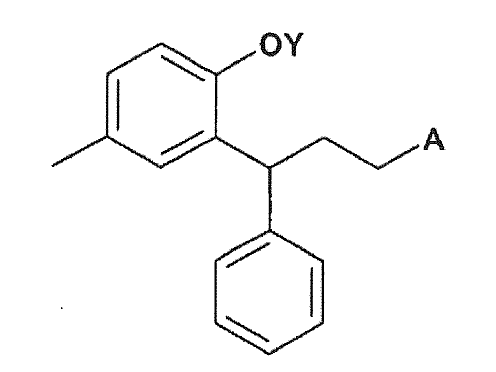 dans laquelle Y peut être : H,
ou Y peut être : COR où
R est sélectionné parmi un groupe alkyle en C₁-C₃
ou Y peut être : P(OR)₂ où
R est sélectionné parmi un groupe alkyle en C₁-C₃ ;
ou Y peut être : PX(Z)₂ où
X est sélectionné parmi O, N-SO₂-C₆H₄-Me, NPh ; et Z est sélectionné parmi OPh, NMe₂ ;
ou Y peut être : Ar-SO₂ où Ar est R'-C₆H₄ où
R' est sélectionné parmi H, un atome d'halogène, NO₂ ;
ou Y peut être : R"-SO₂- où
R" est sélectionné parmi un groupe alkyle en C₂-C₄, un atome d'halogène, NMe₃(CH₂)⁺ ;
et A est I ou Br.

16. Composé sélectionné parmi : le 3-(2-(benzènesulphonyloxy)-5-méthylphényle)-3-phénylpropyl-p-benzènesulphonate ; le 3-(2-éthanesulphonyloxy-5-méthylphényle)-3-phénylpropyl-éthanesulphonate ; la N,N-diisopropyle-3-(2-(benzènesulphonyloxy)-5-méthylphényle)-3-phénylpropylamine, la N,N-diisopropyle-3-(2-éthanesulphonyloxy-5-méthylphényle)-3-phénylpropylamine, l'iodure de 3-(2-(benzènesulphonyloxy)-5-méthylphényle)-3-phénylpropyle, et l'iodure de 3-(2-éthanesulphonyloxy-5-méthylphényle)-3-phénylpropyle.

17. Iodure de 3-(2-(benzènesulphonyloxy)-5-méthylphényle)-3-phénylpropyle, et iodure de 3-(2-éthanesulphonyloxy-5-méthylphényle)-3-phénylpropyle.

18. Utilisation d'iodure de sodium dans le procédé de fabrication de N,N-diisopropyle-3-(2-hydroxy-5-méthylphényle)-3-phénylpropylamine.
